# EUROPEAN PATENT SPECIFICATION

(11) **EP 2 241 606 B1**
(45) Date of publication and mention of the grant of the patent: **15.08.2018**
(21) Application number: 08706682.5
(22) Date of filing: 15.02.2008
(51) Int. Cl.: A61L 27/36

(54) **A METHOD FOR PREPARING A BIOMATERIAL**
VERFAHREN ZUR HERSTELLUNG EINES BIOMATERIALS
PROCÉDÉ DE PRÉPARATION D'UN BIOMATÉRIAU

(30) Priority: 09.01.2008 CN 200810002062
(43) Date of publication of application: 20.10.2010
(60) Divisional application: 18150067.9
(73) Proprietor: Body Organ Biomedical Corp., Taipei City 114 (TW)
(72) Inventor: LAI, Horng-Ji, Taipei 106 Taiwan (CN); LIN, Chien-Cheng, Taipei City 111 Taiwan (CN); LIN, Shang-Ming, Taichung County 420 Taiwan (CN)
(74) Representative: Viering, Jentschura & Partner mbB Patent- und Rechtsanwälte
(86) International application number: PCT/CN2008/070309
(87) International publication number: WO 2009/086743

(56) References cited:
- EP-A1- 0 900 560
- CN-A- 1 727 304
- CN-A- 1 775 950
- CN-A- 1 948 411
- CN-A- 1 986 828
- JP-A- 2004 059 437
- JP-A- 2006 257 014
- US-A1- 2008 128 536
- SANKAR ET AL: "Preparation and partial characterization of collagen sheet from fish (Lates calcarifer) scales", INTERNATIONAL JOURNAL OF BIOLOGICAL MACROMOLECULES, ELSEVIER BV, NL, vol. 42, no. 1, 5 December 2007 (2007-12-05), pages 6-9, XP022375897, ISSN: 0141-8130, DOI: 10.1016/J.IJBIOMAC.2007.08.003

## Description

### BACKGROUND OF THE INVENTION

The present invention relates to a biomaterial and preparation method thereof, and particularly to a biomaterial prepared from fish scales for use in tissue repair and implantation.

Tissue engineering that involves the incorporation of a biomaterial with biologics and/or pharmaceutics and upon implantation in a patient will stimulate angiogenesis, tissue integration, and/or tissue remodeling. The biomaterial is a synthetic and biocompatible material that is used to construct artificial organs, rehabilitation devices, or prostheses and replace natural body tissues.

For over decades, collagen fiber, hydroxyapatite (HAP) and tri-calcium phosphate (TCP) are some biomaterials with great biocompatibility and safety to be used in human tissue implant. However, these biomaterials have disadvantages such as low mechanical strength, risk of chemical residue in cross linking, terrestrial animal transmitted disease.

Therefore, it is desirable to develop a biomaterial having a high mechanical strength, low possibility of contracting with the terrestrial contagious disease and is applicable to tissue repairs or implants. Sankar, et al. discloses a material derived from fish scales that can be used as a wound dressing material as well as a method for preparing said material (Int J Biol Macromol. 2008 Jan 1;42(1):6-9).

### BRIEF SUMMARY OF THE INVENTION

It is an aspect of the invention to provide a method for preparing a tissue repair material from scales, comprising the step of: (i) acellularizing a fish scale to remove most albumin and few glycosaminogylcans after grinding the fish scale; (ii) grinding the fish scale into plurality of ground particles, wherein the ground particles contain a mixture of sponge like matrix and powder; and (iii) cleaning the fish scale until a value of LAL of the tissue repair material is below 1000 Eu/ml.

In some embodiments, the method further comprises a step of extruding the sponge like matrix and powder at a temperature of less than 200 °C or in a desired mold for forming a flaky form, a lump form or a specific form after grinding the fish scale.

In some embodiments, the method further comprises a step of separating the matrix and powder from the ground particles.

It is an aspect of the invention to provide a method for preparing a tissue repair material from scales, comprising the step of: (i) acellularizing a fish scale to remove most albumin and few glycosaminogylcans before grinding the fish scale; (ii) grinding the fish scale into plurality of ground particles, wherein the ground particles contain a mixture of sponge like matrix and powder; and (iii) cleaning the fish scale until a value of LAL of the tissue repair material is below 1000 Eu/ml.

In some embodiments, the method further comprises a step of extruding the sponge like matrix and powder at a temperature of less than 200 °C or in a desired mold for forming a flaky form, a lump form or a specific form after grinding the fish scale.

In some embodiments, the method further comprises a step of separating the matrix and powder from the ground particles.

It is an aspect of the invention to provide a tissue repair material prepared from scale, comprising: a mixture of fibrous tissue and powder prepared by grinding an acellularized fish scale where most albumin and few glycosaminogylcans are removed and the characteristic of the tissue repair material is a LAL value below 1000 Eu/mL.

The biomaterial derived by the methods illustrated above retains the original bonding and the 3D structure of the fish scales, therefore, it has a high mechanical strength, low possibility of contracting with the terrestrial contagious disease and is applicable to repair tissue or tissue implant.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings embodiments which are presently preferred. It should be understood, however, that the invention is not limited to the precise arrangements and instrumentalities shown.

In the drawings:
FIG. 1A is a flow chart illustrating a process for preparing a biomaterial in accordance with the invention.
FIG. 1B is another flow chart illustrating a process for preparing a biomaterial in accordance with the invention.
FIG. 1C is a flow chart illustrating a process for preparing a biomaterial in accordance with examples 1 and 2 of the invention.
FIG. 1D is another flow chart illustrating a process for preparing a biomaterial in accordance with examples 1 and 2 of the invention.
FIG. IE is a flow chart illustrating a process for preparing a biomaterial in accordance with example 3 of the invention.
FIG. IF is another flow chart illustrating a process for preparing a biomaterial in accordance with example 3 of the invention.
FIG. 2A is a SEM (scanning electron microscopy) picture of 3T3 (fibroblast cell) culture with this invention for 5 days.
FIG. 2B is a SEM picture of osteoblastoma culture with this invention for 5 days.
FIG. 3A is a confocal picture of 3T3 (fibroblast cell) culture with this invention for 5 days.
FIG. 3B is a confocal picture of osteoblastoma culture with this invention for 5 days.
FIG. 4 is an H&E stain picture of osteoblastoma cell culture with this invention for 5 days.

### DETAILED DESCRIPTION OF THE INVENTION

Reference will now be made in detail to the present embodiments of the invention, examples of which are illustrated in the accompanying drawings.

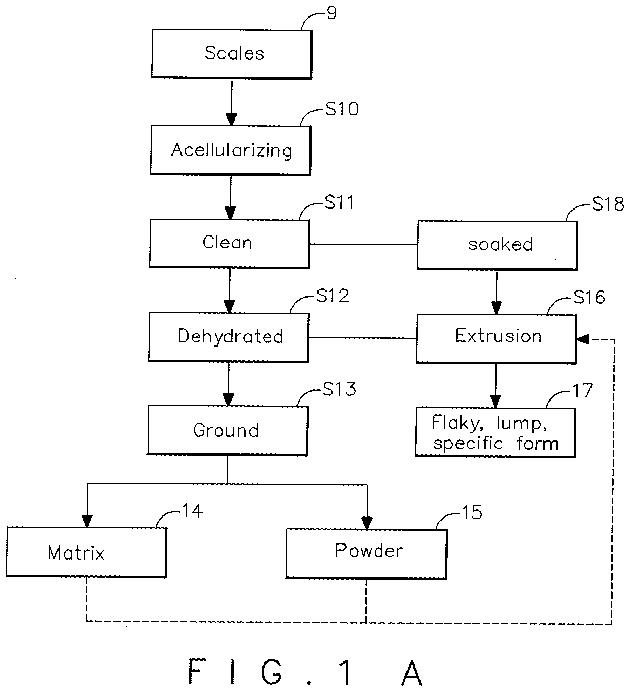

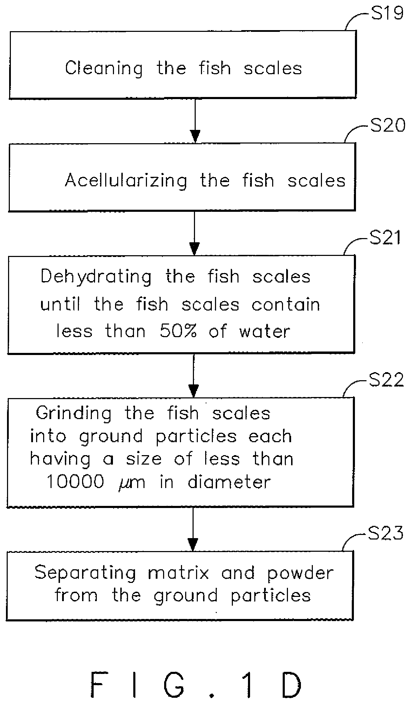

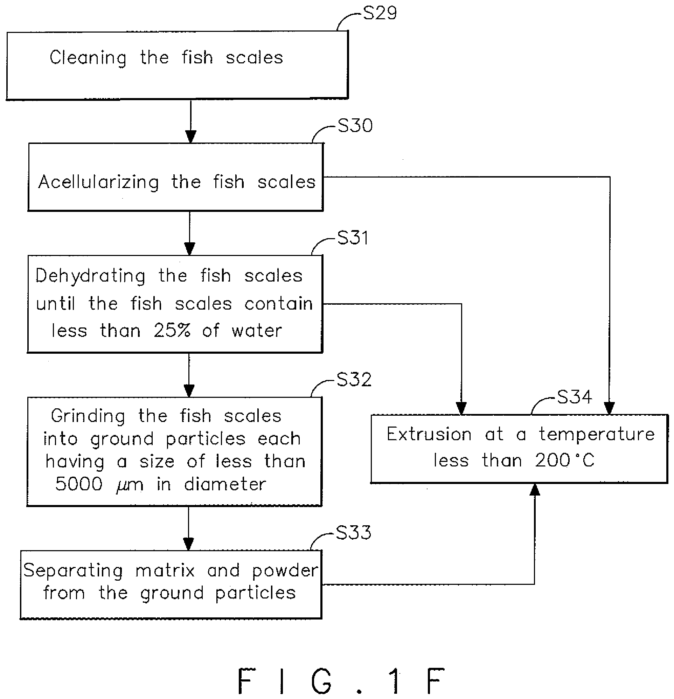

Referring to FIG. 1A, the present invention relates to a biomaterial prepared from fish scales 9. The biomaterial is prepared by a process which comprises the steps of acellularizing the fish scales 9 (S10), dehydrating the fish scales 9 (S12) until the fish scales 9 contain less than about 50% of water. The dehydrated fish scales 9 are then ground (S13) into a ground product having a particle size of less than about 10,000 µm. In an embodiment, the fish scales 9 contain less than about 25% of water, and the ground particles have an average size of less than about 5,000 µm in diameter.

In accordance with some examples of the invention, the fish scales 9 may be freshly provided in a chilled or frozen manner. In a specific example of the invention, the fish scales 9 each having an average size less than about 20 cm in diameter may be selected for preparing the biomaterial. First, the fish scales 9 are acellularized with a reagent such as hypotonic solution, detergent, Triton X-100, SDS (sodium dodecyl sulfate), protease inhibitor, DNase, RNase and so on to remove the cells of the fish scales 9 so as to avoid residual cells affecting the tissue repairs, implants and the biocompatibility. For example, it may cause the host immune response against the biomaterial and the rejection without acellularizing.

Prior to dehydration of the fish scales, the fish scales 9 may be cleaned by washing (S11) in a steam with other cleaning agents including but not limited to surfactant, detergent, warm water and polar solvent such as ethanol at about 60 °C. However, the present invention is not limited to any particular cleaning step. For example, the fish scales are clean enough to pass Limulus Amebocyte Lysate (LAL) test which is an assay for detection and quantitation of bacterial endotoxin. Preferably, the cleaned fish scales would have a LAL test score less than about 1000 Eu/ml.

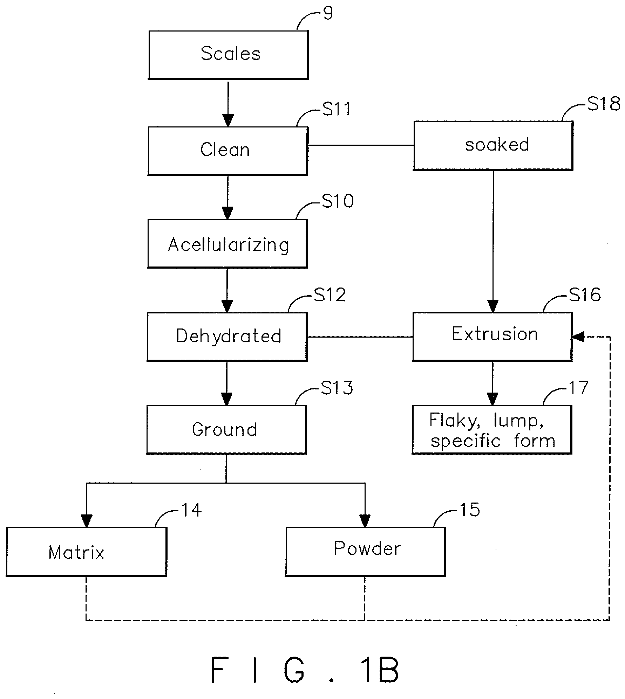

Referring to FIG. 1B, it is another flow chart illustrating a process for preparing a biomaterial in accordance with the invention. The steps of preparing the biomaterial are almost the same with FIG. 1A except that the step of acellularizing the fish scales 9 (S10) is performed after the step of cleaning the fish scales 9 (S11). The sequence of the step of acellularizing the fish scales 9 (S10) and the step of cleaning the fish scales 9 (S11) is optional.

According to one example of the invention, the fish scales 9 is dehydrated (S12) by air spraying, oven, freeze drying or any other conventional dehydration methods available so far. Also, the fish scales can be dehydrated by soaking the fish scales in the ethanol or other polar organic solvent. The fish scales are dehydrated until their water content is less than about 50%, preferably less than about 25%. According to another example of the invention, the dehydrated fish scales may be ground into particles each having an average size of less than about 10,000 µm, preferably about 5000 µm, in diameter. The ground particles may contain a mixture of sponge like matrix 14 and powder 15. For example, the ground particles may be further filtered using an optimal sieve to isolate the matrix and powder. The filtering step may be carried out with an aid of a vibrating means to enhance the sieving effect. As a result, the particles filtered out from the sieve provide the biomaterial in a powder form, whereas the filtrate that remains from the filtering step makes up the biomaterial in a matrix form. Also, the device adopted for grinding the fish scales are not limited to using any particular grinder, grinding machine or any equipment used to reduce the particle size of the fish scales, as long as each of the ground particles has an average size of less than about 10,000 µm, preferably less than about 5000 µm, in diameter.

These products may be further processed, for example, by extrusion (S16), filtering, fully or partially drying and sterilizing to yield sterilized biomaterials. In a preferred embodiment, these steps may be performed with or without heating. These products may be used in combination with a variety of connective tissue repair compositions, or in combination with other active or inactive ingredients.

In one other example, the dehydrated fish scales 9 may be extruded, such as a heat treatment performed at a temperature of less than about 200 °C, to produce the biomaterial in a flaky form, a lump form or extruding in a desired mold for forming a specific form 17. The extrusion process could also be performed under 0°C with the frozen fish scales. However, the present invention is not limited to the above method for producing the biomaterial in the forms illustrated above. For example, the fish scales may also be extruded (S16) at the temperature of less than about 200 °C after the cleaning step (S11) to produce the biomaterial in a flaky form, a lump form or extruding in a desired mold for forming a specific form 17 and it is easy to proceed to the step of extrusion after dehydrated fish scales 9. An additional step (S18) of soaking the fish scales 9 in water is optionally performed before the extrusion process (S16), but after the cleaning step (S11). Moreover, in yet another example of the invention, the biomaterials in the matrix 14 or powder form 15 may be further extruded (S16) at a temperature of less than about 200 °C to convert into the biomaterial in the flaky form, the lump form or extruding in a desired mold for forming the specific form 17. However, the extrusion described above is not limited to use the heat treatment in the present invention. One skilled in the art may also use other heat treatments such as thermal extrusion of any type, thermal pressing and molding steps to produce the biomaterial with a flaky form, a lump form or a specific form.

The biomaterial of the invention contains tissue repair factors and may be manufactured into a tissue repair material for use in repairing a variety of tissue damages and tissue defect sites. For example, the biomaterial of the invention may be prepared for injection or insertion at, into, onto or near bone defect sites, cartilage repair sites, dental alveolar repair site or other soft tissue defect sites. In other examples, the biomaterial may be made as a dressing for transplantation, implantation on surgical grafts or implants to be implanted at, into, onto or near bone defect sites, cartilage repair sites or other tissue defect sites. Accordingly, the invention is also applicable to connective tissue surgical implant with the tissue repair material derived from fish scales, whereby the surgical implant is implanted at a connective tissue defect site.

Summarizing from the above, the invention relates to a biomaterial in powder and/or matrix and/or flaky form prepared from the fish scales 9 for use in a variety of tissue repairs and implantations as well as methods for preparing said biomaterial. These fish scales 9 derived products, also referred to herein as the biomaterial in a powder form, matrix form, flaky form or extruding in a desired mold for forming the specification 17 may contain tissue repair factors and may be further processed to produce a variety of formulations and consistencies.

The invention will now be described in further detail with reference to the following specific examples.

### Example 1: Matrix form

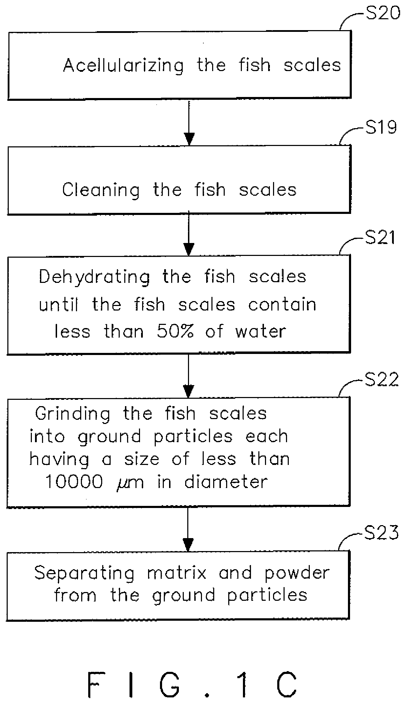

Referring to FIG. 1C, the process for preparing the biomaterial with example 1 in a matrix form from fish scales begins with step (S20), where the fish scales 9 are acellularized S20, then the fish scales are cleaned (S19) with a steam or vapor, for example, until the clean fish scales have a LAL test score of less than about 1000 Eu/ml. Referring to FIG. ID, it is a flow chart illustrating another process for preparing a biomaterial in accordance with example 1 of the invention. First, the fish scales 9 are cleaned (S19), the cleaned fish scales would have a LAL test score less than about 1000 Eu/ml. Then, the fish scales 9 are acellularized (S20). The sequence of the step of acellularizing the fish scales 9 (S20) and the step of cleaning the fish scales 9 (S19) is optional. The process then proceeds to step (S21). In step (S21), the fish scales are dehydrated until the fish scales contain less than 50% of water. Next, in step (S22), the dehydrated fish scales are ground into particles each having an average size of less than 10000 µm in diameter. The ground particles contain a mixture of sponge like matrix and powder. The process proceeds to step (S23). In step (S23), a filtering step is carried out using an optimal sieve for separating the matrix from the mixture. Accordingly, the filtrate from the filtering step makes up the matrix. The matrix contains fibrous tissue that is composed of HAP, TCP and collagen.

### Example 2: Powder form

Referring to FIG. 1C and FIG. ID, they are the flow charts illustrating the process for preparing a biomaterial in accordance with example 2 of the invention, the process for preparing the biomaterial in a powder form is similar to that for the biomaterial in matrix form except the step of filtering (S23) which is performed with a sieve and the biomaterial in grinding particle form include the powder filtered out and matrix left on the sieve after the filtering step (S23) is carried out. Therefore, the powder form differs from the matrix form in that the powder form has a definite or mechanical structure. It is noted that the biomaterial in the present invention does not have performed with enzyme, so it retains not only the collagen, HAP, but also the original bonding and the 3D structure of the fish scale. The powder form is different from the matrix form and the powder size is less than 5000µm in diameter.

### Example 3: Flaky form, a lump form or extruding in a desired mold for forming a specific form.

Referring to FIG. IE, it is a flow chart illustrating a process for preparing a biomaterial in accordance with example 3 of the invention to produce the biomaterial in a flaky form, a lump form or extruding in a desired mold for forming a specific form. The process for preparing the biomaterial from fish scales begins with step (S30), where the fish scales are acellularized, then the fish scales are cleaned (S29) until the clean fish scales have a LAL test score of less than about 1000 Eu/ml.

Referring to FIG. IF, it is another flow chart illustrating a process for preparing a biomaterial in accordance with example 3 of the invention to produce the biomaterial in a flaky form, a lump form or extruding in a desired mold for forming a specific form. The process for preparing the biomaterial from fish scales begins with step (S29), where the fish scales are cleaned (S29) until the clean fish scales have a LAL test score of less than about 1000 Eu/ml, then the fish scales are acellularized (S30).The sequence of the step of acellularizing the fish scales 9 (S30) and the step of cleaning the fish scales 9 (S29) is optional.

The process then proceeds to step (S31). In step (S31), the fish scales are dehydrated until the fish scales contain less than 25% of water. Next, in step (S32), the dehydrated fish scales are ground into particles each having an average size of less than 5000µm in diameter. The ground particles contain a mixture of sponge like matrix and powder. The process proceeds to step (S33). In step (S33), a filtering step is carried out using an optimal sieve for separating the matrix and powder from the mixture. The process proceeds to step (S34) which includes extruding the matrix and powder. The matrix and powder can be respectively performed in the step of extruding. That is, in the step of extruding, the matrix and powder can be combined together. Besides, the matrix and powder can be combined together with different percentage for different purpose, such as bone repair or skin repair.

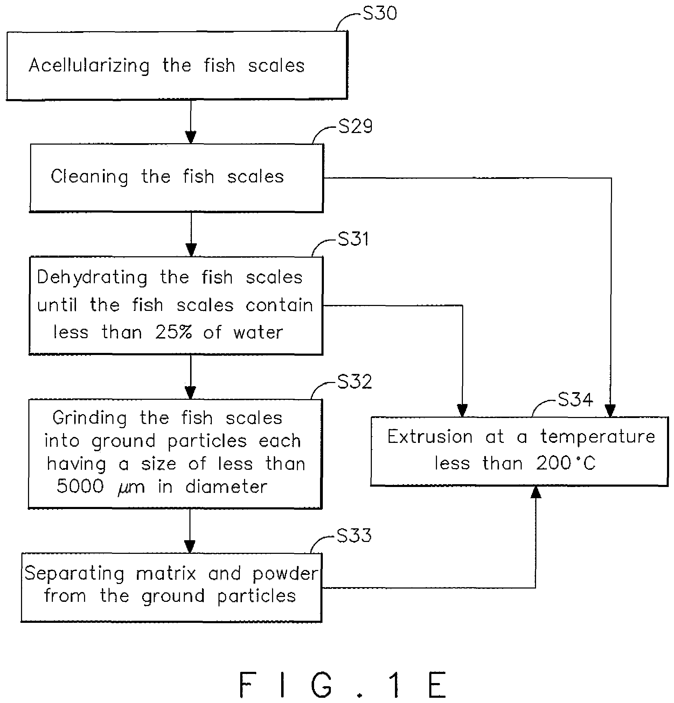

Alternatively, the step (S34) can be performed directly after the cleaning step (S29) is performed to simplify the overall process in the process for preparing the biomaterial. Also, the process may include the cleaning and dehydration steps before performing the extrusion step on the cleaned and dehydrated fish scales as shown in FIG. 1E and FIG. IF.

Accordingly, the dehydrated fish scales, cleaned fish scales without dehydration, or the biomaterials derived from the fish scales in matrix or powder form are extruded to produce the biomaterial in a flaky form, a lump form or extruding in a desired mold for forming a specific form. The biomaterial derived from whole fish scale or fish scales with different types, previously cold pressed with a pressure of more than 100g in 2.5 cm³, preferably, more than 1 kg in 2.5 cm³, are submitted to hot pressing performed at a temperature of less than about 200 °C in a desired mold.

The temperature in the step of extruding just gives an example, not limited. For example, the fish scales can be extruded during -18°C to 4°C, 30°C to 60°C, less than about 200 °C. Further, the time of extrusion process is different with different pressure. The cross linking of the biomaterials can be achieved physically by heating or chemically by adding with a cross linker such as glutaraldehyde, EDC (1-Ethyl-3-(3-dimethylaminopropyl)-carbodiimide) at an optimal concentration before extrusion is performed. The cross linker is reactive with the amines group or other reactive group in the biomaterials.

During the step of acellularizing the fish scales, only the most albumin and few glycosaminoglycans are removed, the collagen, elastin and most of the glycosaminoglycans are residual in the extracellular matrix of the original structure, therefore, the acellularized biomaterial can supply the structure for cells to move in and has good biocompatibility.

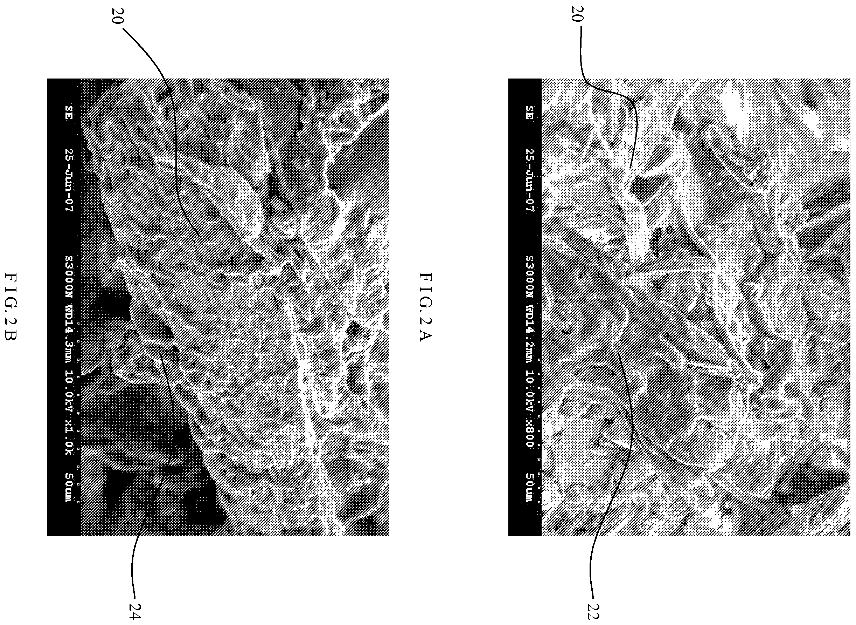

FIG. 2A is a SEM picture of 3T3 (fibroblast cell 22) culture with the biomaterial 20 described in present invention for 5 days. FIG. 2B is a SEM picture of osteoblastoma 24 culture with the biomaterial 20 described in the present invention for 5 days. These pictures show that cells (fibroblast 22 or osteoblastoma 24) can grow well on the biomaterial 20 described in the present invention.

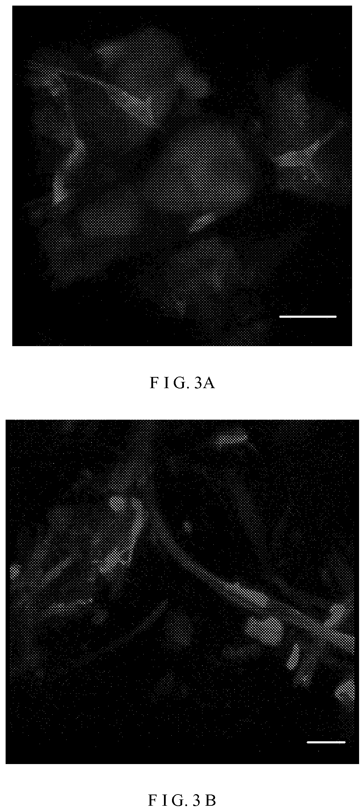

FIG. 3A is a confocal picture of 3T3 (fibroblast cell) culture with this invention for 5 days. FIG. 3B is a confocal picture of osteoblastoma culture with this invention for 5 days. These pictures show that cells (fibroblast or osteoblastoma) can grow well on this invention.

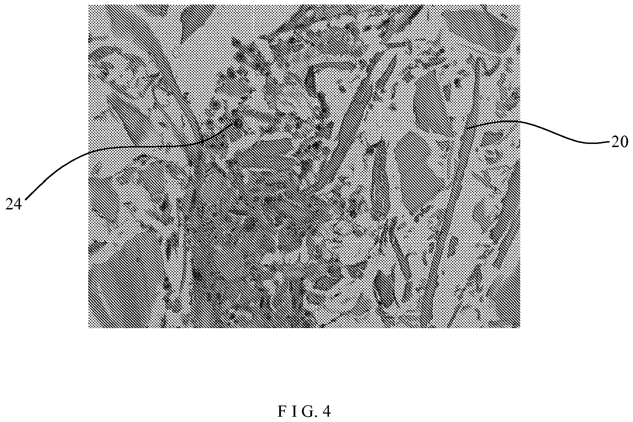

FIG. 4 is an H&E stain picture of osteoblastoma cell 24 culture with the biomaterial 20 described in the present invention for 5 days. These pictures show that osteoblastoma cell 24 can growth well on the biomaterial 20 described in the present invention.

## Claims

1. A method for preparing a tissue repair material_from scales, comprising the step of:
acellularizing a fish scale to remove most albumin and few glycosaminogylcans_after grinding the fish scale;
grinding the fish scale into plurality of ground particles, wherein the ground particles contain a mixture of sponge like matrix and powder; and
cleaning the fish scale until a value of LAL of the tissue repair material is below 1000 Eu/ml.

2. The method according to claim 1, further comprising a step of extruding the sponge like matrix and powder at a temperature of less than 200 °C or in a desired mold for forming a flaky form, a lump form or a specific form after grinding the fish scale.

3. The method according to claim 1, further comprising a step of separating the matrix and powder from the ground particles.

4. A tissue repair material_prepared from scale, comprising:
a mixture of fibrous tissue and powder prepared by grinding an acellularized fish scale where most albumin and few glycosaminogylcans are removed_and the characteristic of the tissue repair material is a LAL value below 1000 Eu/mL.

5. A method for preparing a tissue repair material from scales, comprising the step of:
acellularizing a fish scale to remove most albumin and few glycosaminogylcans before grinding the fish scale;
grinding the fish scale into plurality of ground particles, wherein the ground particles contain a mixture of sponge like matrix and powder; and
cleaning the fish scale until a value of LAL of the tissue repair material is below 1000 Eu/ml.

6. The method according to claim 5, further comprising a step of extruding the sponge like matrix and powder at a temperature of less than 200 °C or in a desired mold for forming a flaky form, a lump form or a specific form after grinding the fish scale.

7. The method according to claim 5, further comprising a step of separating the matrix and powder from the ground particles.

## Patentansprüche

1. Ein Verfahren zum Herstellen eines Gewebereparatur-Materials aus Schuppen, umfassend den Schritt des:
Azellularisierens einer Fischschuppe, um das meiste Albumin und wenige Glykosaminoglykane nach dem Mahlen der Fischschuppe zu entfernen;
Mahlens der Fischschuppe in eine Vielzahl von gemahlenen Teilchen, wobei die gemahlenen Teilchen eine Mischung aus schwammähnlicher Matrix und Pulver enthalten; und
Reinigens der Fischschuppe bis ein LAL-Wert des Gewebereparatur-Materials unter 1000 Eu/mL vorliegt.

2. Das Verfahren gemäß Anspruch 1, ferner umfassend einen Schritt des Extrudierens der schwammähnlichen Matrix und des Pulvers bei einer Temperatur von weniger als 200 °C oder in eine gewünschte Form zum Bilden einer flockigen Form, einer Klumpenform oder einer spezifischen Form nach dem Mahlen der Fischschuppe.

3. Das Verfahren gemäß Anspruch 1, ferner umfassend einen Schritt des Trennens der Matrix und des Pulvers von den gemahlenen Teilchen.

4. Ein Gewebereparatur-Material, hergestellt aus einer Schuppe, umfassend:
eine Mischung aus fibrösem Gewebe und Pulver, das durch Mahlen einer azellularisierten Fischschuppe hergestellt wurde, wobei das meiste Albumin und wenige Glykosaminoglykane entfernt werden und wobei die Eigenschaft des Gewebereparatur-Materials ein LAL-Wert unter 1000 Eu/mL ist.

5. Ein Verfahren zum Herstellen eines Gewebereparatur-Materials aus Schuppen, umfassend den Schritt des:
Azellularisierens einer Fischschuppe, um das meiste Albumin und wenige Glykosaminoglykane vor dem Mahlen der Fischschuppe zu entfernen;
Mahlens der Fischschuppe in eine Vielzahl von gemahlenen Teilchen, wobei die gemahlenen Teilchen eine Mischung aus schwammähnlicher Matrix und Pulver enthalten; und
Reinigens der Fischschuppe bis ein LAL-Wert des Gewebereparatur-Materials unter 1000 Eu/mL vorliegt.

6. Das Verfahren gemäß Anspruch 5, ferner umfassend einen Schritt des Extrudierens der schwammähnlichen Matrix und des Pulvers bei einer Temperatur von weniger als 200 °C oder in eine gewünschte Form zum Bilden einer flockigen Form, einer Klumpenform oder einer spezifischen Form, nach dem Mahlen der Fischschuppe.

7. Das Verfahren gemäß Anspruch 5, ferner umfassend einen Schritt des Trennens der Matrix und des Pulvers von den gemahlenen Teilchen.

## Revendications

1. Procédé de préparation d'un matériau de réparation tissulaire à partir d'écailles, comprenant l'étape de :
élimination des cellules d'une écaille de poisson pour éliminer la majeure partie de l'albumine et quelques glycosaminoglycanes après le broyage de l'écaille de poisson ;
broyage de l'écaille de poisson en une pluralité de particules broyées, dans lequel les particules broyées contiennent un mélange de matrice de type éponge et de poudre ; et
nettoyage de l'écaille de poisson jusqu'à ce qu'une valeur de LAL du matériau de réparation tissulaire soit inférieure à 1 000 Eu/ml

2. Procédé selon la revendication 1, comprenant en outre une étape d'extrusion de la matrice de type éponge et de la poudre à une température inférieure à 200 °C ou dans un moule souhaité pour former une forme en lamelles, une forme en blocs ou une forme spécifique après le broyage de l'écaille de poisson.

3. Procédé selon la revendication 1, comprenant en outre une étape de séparation de la matrice et de la poudre des particules broyées.

4. Matériau de réparation tissulaire préparé à partir d'écaille, comprenant :
un mélange de tissu fibreux et de poudre préparé par broyage d'une écaille de poisson ayant subi une élimination des cellules où la majeure partie de l'albumine et quelques glycosaminoglycanes sont éliminés et la caractéristique du matériau de réparation tissulaire est une valeur de LAL inférieure à 1 000 Eu/ml.

5. Procédé de préparation d'un matériau de réparation tissulaire à partir d'écailles, comprenant l'étape de :
élimination des cellules d'une écaille de poisson pour éliminer la majeure partie de l'albumine et quelques glycosaminoglycanes avant le broyage de l'écaille de poisson ;
broyage de l'écaille de poisson en une pluralité de particules broyées, dans lequel les particules broyées contiennent un mélange de matrice de type éponge et de poudre ; et
nettoyage de l'écaille de poisson jusqu'à ce qu'une valeur de LAL du matériau de réparation tissulaire soit inférieure à 1 000 Eu/ml.

6. Procédé selon la revendication 5, comprenant en outre une étape d'extrusion de la matrice de type éponge et de la poudre à une température inférieure à 200 °C ou dans un moule souhaité pour former une forme en lamelles, une forme en blocs ou une forme spécifique après le broyage de l'écaille de poisson.

7. Procédé selon la revendication 5, comprenant en outre une étape de séparation de la matrice et de la poudre des particules broyées.
